# EUROPÄISCHE PATENTSCHRIFT

(11) **EP 2 496 935 B1**
(45) Veröffentlichungstag und Bekanntmachung des Hinweises auf die Patenterteilung: **12.11.2014**
(21) Anmeldenummer: 10754752.3
(22) Anmeldetag: 20.09.2010
(51) Int. Cl.: G01N 27/414, G01N 33/00

(54) **Verfahren zur Herstellung eines Gassensors**
Method of fabricating a gas sensor
Procédé de fabrication d'un capteur à gaz

(30) Priorität: 03.11.2009 DE 102009046317
(43) Veröffentlichungstag der Anmeldung: 12.09.2012
(73) Patentinhaber: Robert Bosch GmbH, 70442 Stuttgart (DE)
(72) Erfinder: NEFF, Petra, 70567 Stuttgart (DE); MARTIN, Alexander, 71642 Ludwigsburg (DE)
(86) Internationale Anmeldenummer: PCT/EP2010/063763
(87) Internationale Veröffentlichungsnummer: WO 2011/054577

(56) Entgegenhaltungen:
- DE-A1- 2 918 932
- DE-A1- 10 245 614
- DE-A1- 19 916 798
- DE-A1-102004 047 797
- DE-A1-102009 000 820
- US-A- 5 698 771

## Beschreibung

### Stand der Technik

Die vorliegende Erfindung betrifft einen Sensor zum Detektieren wenigstens eines ersten Mediums in einem Mediengemisch aus wenigstens dem ersten und einem zweiten Medium, ein Verfahren zum Herstellen des Sensors sowie einen Chip mit dem Sensor.

Beispielsweise werden zur Bestimmung von Gaskomponenten in Gasgemischen unter anderem Sensoren in Form von Feldeffekttransistoren eingesetzt. Dabei reagiert beispielsweise eine Gate-Elektrode des Feldeffekttransistors sensitiv auf die zu bestimmenden Gaskomponenten, wodurch es zu einer Veränderung des Potentials der Gate-Elektrode kommt. Eine sich dadurch ergebende Veränderung des Stromflusses zwischen einer Source- und Drain-Elektrode des Feldeffekttransistors wird der Konzentration einer Gaskomponente zugeordnet. Derartige Sensoren werden als ChemFETs bezeichnet. Solche ChemFETs werden insbesondere in Abgasleitungen von Verbrennungsmotoren eingesetzt, um beispielsweise den Anteil an Stickoxid (nachfolgend als NOₓ bezeichnet) im Abgas zu messen, wie in der DE 10 2007 040 726 A1 und in der Zwischenveröffentlichung DE 10 2009000820 A1 beschrieben.

Weiterhin werden auch Festelektrolyt-Gassensoren typischerweise für Messungen an Abgasen eingesetzt. In der DE 2918932 wird ein solcher Sensor beschrieben, bei dem eine der Elektroden mit einem in einem Aluminiumoxidträger vorgesehenen Platinkatalysator abgedeckt ist.

### Vorteile der Erfindung

Das in Anspruch 1 definierte, erfindungsgemäße Verfahren bietet gegenüber herkömmlichen Lösungen den Vorteil, dass der Katalysator, dessen Wärmeausdehnungskoeffizient (nachfolgend als WAK bezeichnet) sich im Allgemeinen von dem der Elektrode unterscheidet, beabstandet von der Elektrode auf und/oder in der durchgängig porösen Trägerkeramik angeordnet ist und es daher zu keiner thermischen Verspannung zwischen dem Katalysator und der Elektrode kommt, wenn diese stark variierenden Temperaturen, wie typischerweise in der Abgasleitung eines Verbrennungsmotors der Fall, ausgesetzt sind. Außerdem haftet der Katalysator vergleichsweise gut auf der porösen Trägerkeramik wegen derer hohen Oberflächenrauigkeit. Weiterhin wird - aufgrund des Abstands - eine katalytische Aktivität der Elektrode nicht durch den Katalysator beeinflusst, wodurch die Messgenauigkeit des Sensors verbessert wird.

Die in den Unteransprüchen aufgeführten Merkmale beziehen sich auf vorteilhafte Weiterbildungen und Verbesserungen des Gegenstands der Erfindung.

### Zeichnungen

Ausführungsbeispiele der Erfindung sind in der Zeichnung dargestellt und in der nachfolgenden Beschreibung näher erläutert.

Es zeigen
- Fig. 1: in einer Schnittansicht einen erfindungsgemäß hergestellten Sensor;
- Fig. 2: in einer Schnittansicht einen nicht erfindungsgemäß hergestellten Sensor; und
- Fig. 3: in einer Draufsicht einen Chip.

### Beschreibung von Ausführungsbeispielen

In den Figuren bezeichnen gleiche Bezugszeichen gleiche oder funktionsgleiche Elemente, soweit nichts Gegenteiliges angegeben ist.

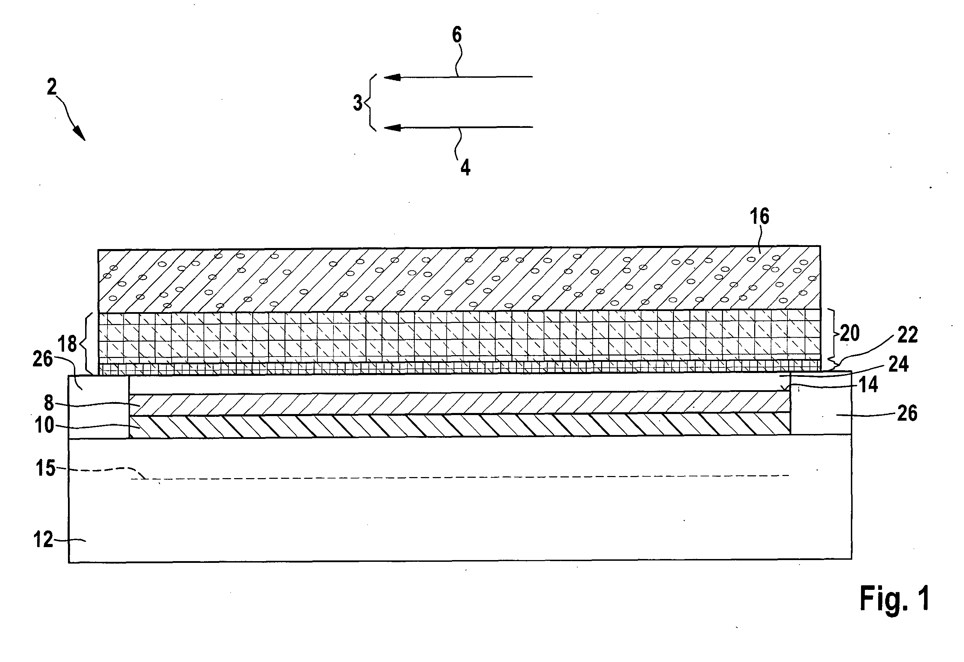

Fig. 1 zeigt einen Sensor 2 in einer Schnittansicht.

Gemäß dem Ausführungsbeispiel ist der Sensor 2 in oder angrenzend an einen Abgasstrom 3 in einer nicht weiter dargestellten Abgasleitung eines Verbrennungsmotors angeordnet.

Der Abgasstrom 3 weist ein Gasgemisch aus einem ersten Gas 4 und einem zweiten Gas 6 auf. Gemäß dem Ausführungsbeispiel ist das erste Gas NOₓ-Gas, das zweite Gas 6 Kohlenwasserstoffgas. Selbstverständlich kann der Abgasstrom 3 auch noch weitere Gase enthalten.

Mittels des Sensors 2 soll das NOₓ-Gas 4 in dem Abgasstrom 3 detektiert werden. Mit "Detektieren" ist vorliegend sowohl das reine Erkennen des Vorliegens von NOₓ-Gas 4 in dem Abgasstrom gemeint als auch vorzugsweise die mengenmäßige Erfassung des NOₓ Gases 4. Dazu ist der Sensor 2 gemäß diesem Ausführungsbeispiel wie folgt ausgebildet:
Der Sensor 2 ist in Form eines ChemFET ausgeführt und weist eine Gate-Elektrode 8 auf, welche über eine Isolationsschicht 10 mit einem Halbleitersubstrat 12 in Kontakt steht.

Das Halbleitersubstrat 12 kann aus Galliumnitrid, Aluminiumnitrid, Gallium-Aluminiumnitrid oder Siliziumcarbid ausgeführt sein. Gemäß diesem Ausführungsbeispiel ist sie als Siliziumcarbid ausgeführt.

Die Gate-Elektrode 8 ist vorzugsweise aus einem oxidationsstabilen Edelmetall, wie Platin, Palladium, Gold, Iridium, Rhenium, Rhodium oder Mischungen derselben und/oder aus Zumischung von Elementverbindungen unedler Metalle, wie Hafnium, Tantal und/oder Aluminium ausgebildet.

Die Gate-Elektrode 8 kann ferner eine poröse Beschichtung 14 aus einem Edelmetall oder aus einem Edelmetall-Metalloxid-Mischmaterial aufweisen. Das Material für die poröse Beschichtung 14 ist vorzugsweise ausgewählt aus den Nebengruppenelementen wie Hafnium, Tantal, Niobium, Tantal, Molybdän, Rhodium, Platin, Palladium, Silber, Gold oder Mischungen derselben. Ferner kommen elektrisch leitfähige Verbindungen wie Nitride, Karbide oder Silizide der Nebengruppenelemente wie beispielsweise Wolfram- oder Tantalsilizid in Frage. Als Edelmetall-Metalloxid-Mischmaterial eignen sich insbesondere Cermets, welche neben den genannten Nebengruppenelementen bzw. deren Karbiden oder Siliziden eine keramische Komponente wie beispielsweise Aluminiumoxid, Siliziumdioxid, Zirkoniumdioxid, selten Erdmetalle, wie insbesondere Magnesiumoxid, aufweisen.

Die die poröse Beschichtung 14 vorzugsweise umfassende Elektrode 8 ist dazu eingerichtet, ihr elektrisches Potential zu ändern, wenn diese mit dem NOₓ-Gas 4 oder dem Kohlenwasserstoffgas 6 in dem Abgasstrom 3 oder beiden Gasen in Berührung kommt. Neben der Gate-Elektrode 8 weist der Sensor 2 nicht dargestellte Source- und Drain-Elektroden auf, wobei eine Änderung des elektrischen Potentials der Gate-Elektrode 8 zu einer Veränderung eines Stroms in einem Kanalbereich 15 in dem Halbleitersubstrat 12 zwischen der Source- und der Drainelektrode führt. Die Veränderung des Stroms wird in einer nicht dargestellten Auswerteeinheit ausgewertet, um festzustellen, welches Gas und in welcher Menge dieses an der Elektrode 8 vorliegt.

Da aber gemäß dem Ausführungsbeispiel nur das NOₓ-Gas detektiert werden soll, ist ein Katalysator 16 zwischen der Elektrode 8 und dem Abgasstrom 3 angeordnet. Der Katalysator 16 dient der Eliminierung einzelner Gaskomponenten in dem Abgasstrom 3, zu denen die Elektrode 8 eine unerwünschte Querempfindlichkeit aufweist. Dabei werden diese unerwünschten Gaskomponenten von dem Katalysator 16 zu Gaskomponenten umgesetzt, die eine Messung der zu bestimmenden Gaskomponente nicht behindern. Gemäß dem vorliegenden Ausführungsbeispiel ist der Katalysator 16 daher als Oxidationskatalysator ausgebildet, welcher das Kohlenwasserstoffgas 6 zu Kohlendioxid und Wasser oxidiert. Der katalytische Oxidationsprozess läuft vorzugsweise vorwiegend bei einer Temperatur des Abgasstroms 3 im Bereich zwischen 100 bis 650°, bevorzugt zwischen 250 und 550° ab. Die Reaktionsprodukte Kohlendioxid und Wasser haben keinen wesentlichen Einfluss auf das elektrische Potential der Elektrode 8. Das NOₓ-Gas 4 dagegen kann ungehindert durch den Oxidationskatalysator 16 hindurch zu der Elektrode 8 gelangen, wo es zu einer Änderung deren elektrischen Potentials beiträgt.

Gemäß dem Ausführungsbeispiel ist der Katalysator 16 als eine Vielzahl von Partikeln ausgebildet sein, welche jeweils einen keramischen Kern, beispielsweise aus Aluminiumoxid oder Zirkonoxid, aufweisen, der eine katalytische Ummantelung insbesondere aus Edelmetall, wie Platin, Rhodium, Palladium, Iridium oder Mischungen derselben, trägt. Alternativ kann der Katalysator 16 selbst als poröse Schicht insbesondere aus einem der vorgenannten Edelmetalle ausgebildet sein.

Der Katalysator 16 ist gemäß dem Ausführungsbeispiel teilweise auf, d. h. abgasstromseitig, und teilweise innerhalb einer als Schicht ausgebildeten, durchgängig porösen Trägerkeramik 18 angeordnet, welche selbst zwischen der Elektrode 8 und dem Abgasstrom 13 angeordnet ist. Die Trägerkeramik 18 besteht vorzugsweise aus Silizium oder Siliziumkarbid. Die Trägerkeramik 18 weist in einem abgasstromseitigen Bereich 20 Poren mit einem ersten Durchmesser von vorzugsweise zwischen 2 und 20 µm auf, in welchen der Katalysator 16 in Partikelform aufgenommen ist. Die Trägerkeramik 18 weist ferner einen elektrodenseitigen Bereich 22 auf, welcher mit Poren ausgebildet ist, die einen zweiten Durchmesser von etwa 0,2-2 µm oder kleiner aufweisen. In diese Poren bzw. durch diese Poren mit dem zweiten Durchmesser kann der Katalysator 16 nicht gelangen, so dass die Katalysatorpartikel 16 nicht ungewollt mit der Elektrode 8 in Kontakt kommen können bzw. durch die Trägerkeramik 18 hindurch fallen können.

Alternativ kann der Katalysator 16 auch als poröse Schicht ausgebildet sein, welche auf der Trägerkeramik 18 angeordnet ist.

Erfindungsgemäß ist die Trägerkeramik 18 durch einen Spalt 24 von der Elektrode 8 beabstandet angeordnet. Dadurch wird vermieden, dass sich die Trägerkeramik 18 und die Elektrode 8 direkt berühren, so dass ein Verspannen dieser zueinander, beispielsweise aufgrund unterschiedlicher WAKs, vermieden wird.

Ferner weist der Sensor 2 eine Passivierung 26 auf, welche zusammen mit dem Katalysator 16, der Trägerkeramik 18 und dem Halbleitersubstrat 12 die Elektrode 8 umgibt. Die Passivierung 26 ist gasdicht ausgebildet und vorzugsweise aus Siliziumdioxid und/oder Siliziumnitrid ausgeführt.

Vorteilhaft sind das Halbleitersubstrat 12 und die Trägerkeramik 18 aus demselben Material oder unterschiedlichen Materialien mit im Wesentlich denselben WAKs ausgebildet. Vorzugsweise sind beispielsweise das Halbleitersubstrat 12 und die Trägerkeramik 18 beide aus Siliziumkarbid ausgebildet. Dadurch kann vermieden werden, dass es zu Verspannungen zwischen der Trägerkeramik 18 und dem Halbleitersubstrat 12 kommt; es wird also ein großer Kraftfluss durch die Bauteilpassivierung 26 vermieden. Die Bauteilpassivierung 26 und die Isolationsschicht 10 sind derart dünn ausgeführt (Fig. 1 stellt die Größenverhältnisse nicht realitätsgetreu dar), dass diese für eine Verspannung der Komponenten des Sensors 2 zueinander nicht relevant sind.

Nachfolgend wird das erfindungsgemäße Verfahren zum Herstellen des Sensors 2 gemäß dem Ausführungsbeispiel nach Fig. 1 näher erläutert.

Zunächst wird das Halbleitersubstrat 12 bereitgestellt und auf diesem die Isolationsschicht 10 aufgebracht. Anschließend wird auf die Isolationsschicht 10 die Elektrode 8 aufgebracht. Vor oder nach dem Aufbringen der Isolationsschicht 10 oder der Elektrode 8 wird eine Passivierung 26 auf das Halbleitersubstrat 12 aufgebracht.

In einem weiteren Schritt wird auf die Elektrode 8 eine Schutzschicht, beispielsweise ein Fotoschutzlack, aufgebracht. Hiernach wird die Trägerkeramik 18 auf der Schutzschicht erzeugt, insbesondere abgeschieden. Die Trägerkeramik 18 wird beispielsweise derart abgeschieden, dass sie die bereits oberhalb beschriebenen durchgängigen Poren aufweist. Andererseits ist es auch möglich, die Trägerkeramik 18 zunächst auf der Schutzschicht zu erzeugen und hiernach die Poren in der Trägerkeramik 18 zu erzeugen.

In einem weiteren Schritt wird der Katalysator 16 auf die Trägerkeramik 18 aufgebracht und wenigstens teilweise in diese eingebracht.

Gemäß einem nicht dargestellten Ausführungsbeispiel kann der Katalysator 16 als separate Schicht auf die Trägerkeramik 18 abgasstromseitig aufgebracht werden. Gemäß dem vorliegenden Ausführungsbeispiel aber wird der Katalysator 16 in der bereits oberhalb beschriebenen Partikelform auf die Trägerkeramik 18 aufgebracht und in den abgasstromseitigen Bereich 20 der Trägerkeramik 18 eingebracht. Dies kann mittels Tauchens der Trägerkeramik 18 in eine Suspension des Katalysators 16 oder Aufsprühen einer solchen Suspension auf die Trägerkeramik 18 oder auch mittels Verdruckens einer Katalysatorpaste im Siebdruckverfahren auf die Trägerkeramik 18 geschehen. All diesen Verfahren ist gemeinsam, dass ein gewisser Anteil der Katalysatorpartikel 16 in die Poren des abgasstromseitigen Bereichs 20 der Trägerkeramik 18 eingelagert werden. Dadurch, dass die Poren in dem Bereich 22 einen kleineren Durchmesser aufweisen als die Katalysatorpartikel 16, wird verhindert, dass die Katalysatorpartikel 16 zu der Schutzschicht oder später zu der Elektrode 8 gelangen können.

In einem weiteren Schritt wird die Anordnung bestehend wenigstens aus dem Katalysator 16 und der Trägerkeramik 18 wärmebehandelt oder gesintert, um die Haftung zwischen dem Katalysator 16 und der Trägerkeramik 18 zu erhöhen. Eine derartige Wärmebehandlung kann beispielsweise bei einer Temperatur zwischen in etwa 200 und 600° erfolgen.

Es ist auch denkbar, die Trägerkeramik 18 zunächst zu erzeugen und mit dem Katalysator 16 zu beschichten und die so geschaffene Anordnung gegebenenfalls Wärme zu behandeln oder zu sintern. In einem weiteren Schritt könnte diese Anordnung auf eine weitere Anordnung bestehend wenigstens aus dem Halbleitersubstrat 12, der Isolationsschicht 10 und der Elektrode 8 auf letztere aufgebracht werden.

Nach dem Aufbringen des Katalysators 16 oder vorzugsweise nach der Wärmebehandlung, Sinterung oder Porofizierung der Trägerkeramik 18 wird die Schutzschicht entfernt, so dass der freie Spalt 24 zwischen der Trägerkeramik 18 und der Elektrode 8 entsteht.

Um die Schutzschicht zu entfernen, stehen verschiedene Möglichkeiten offen. Gemeinsam soll diesen Möglichkeiten sein, dass die Schutzschicht durch die Poren in der Trägerkeramik 18 aus dem Spalt 24 entfernt wird.

In dem Fall, dass die Schutzschicht als Schutzlack ausgebildet ist, kann dieser durch ein organisches Lösungsmittel, in dem der Schutzlack gut lösbar ist, entfernt werden. Dazu wird das organische Lösungsmittel durch die Poren in der Trägerkeramik 18 hindurch der Schutzschicht zugeführt, welche sich dadurch auflöst und durch die Poren ausgeschwemmt wird. Gemäß einer anderen Variante kann die Entfernung der Schutzschicht durch thermisches Erhitzen entfernt werden, wobei die Schutzschicht zunächst thermisch zersetzt wird und hiernach durch die Poren in der Trägerkeramik 18 ausdampft. Für dieses Verfahren ist es günstig, wenn die Schutzschicht aus einem thermisch zersetzbaren Photopolymer ausgebildet wird. Gemäß einem vorteilhaften Ausführungsbeispiel wird mit derselben Wärmebehandlung die Schutzschicht aus dem Spalt 24 entfernt und gleichzeitig die Haftung zwischen dem Katalysator 16 und der Trägerkeramik 18 erhöht. Die vorstehend beschriebene separate Wärmebehandlung des Katalysators 16 samt der Trägerkeramik 18 entfällt damit, weshalb das Herstellungsverfahren zum Herstellen des Sensors 2 insgesamt vereinfacht wird.

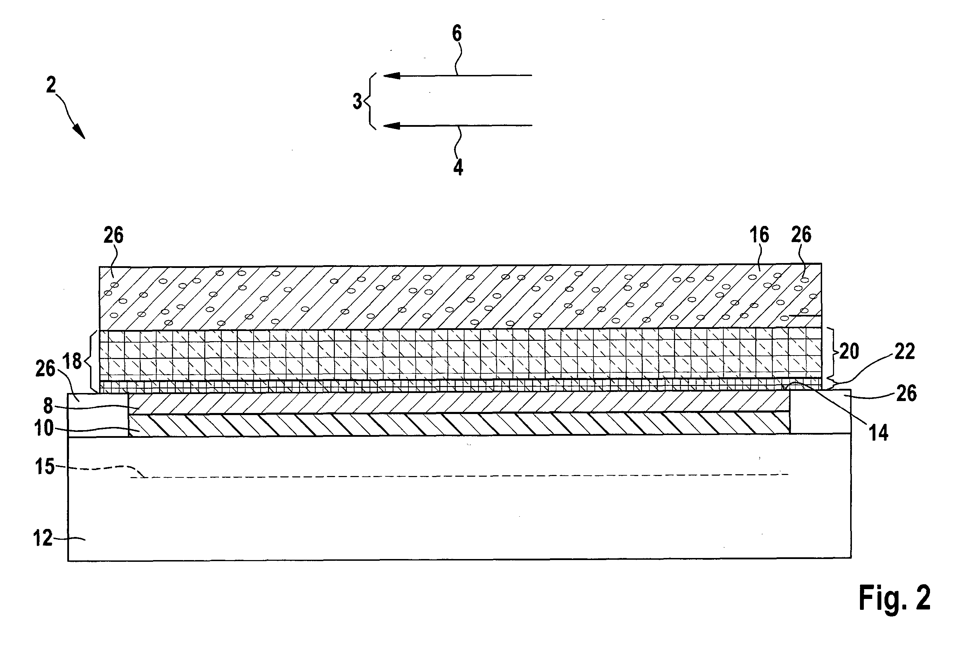

Der Sensor 2 im nicht erfindungsgemäßen Ausführungsbeispiel gemäß Fig. 2 unterscheidet sich von dem gemäß Fig. 1 dadurch, dass bei dem Ausführungsbeispiel gemäß Fig. 2 die Trägerkeramik 18 direkt auf der Elektrode 8, welche gegebenenfalls die Beschichtung 14 umfasst, aufgebracht wird. Demnach entfällt bei dem Verfahren zum Herstellen des Sensors 2 gemäß dem Ausführungsbeispiel gemäß Fig. 2 der oberhalb im Zusammenhang mit Fig. 1 beschriebene Verfahrensschritt, wonach die Schutzschicht auf die Elektrode 8 aufgebracht wird und diese später wieder entfernt wird. Im Übrigen sind die beschriebenen Verfahren zum Herstellen des Sensors nach Fig. 1 und des Sensors 2 nach Fig. 2 identisch.

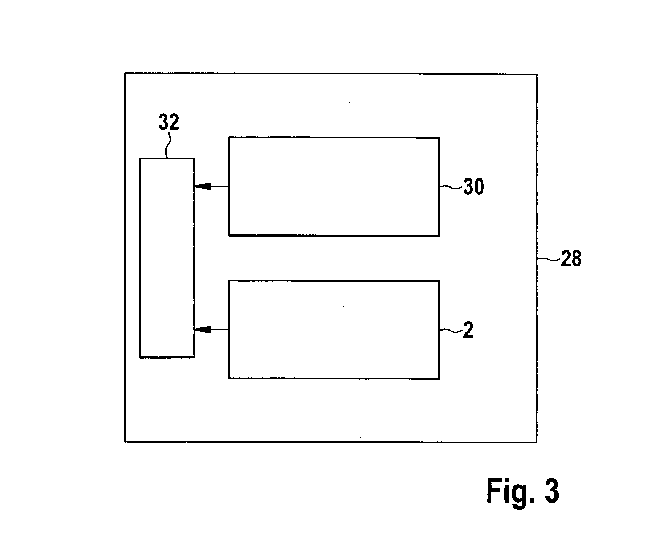

Fig. 3 zeigt in einer Draufsicht einen Chip 28, welcher den Sensor 2 gemäß dem Ausführungsbeispiel nach Fig. 1 oder 2 aufweist. Der Chip 28 weist ferner einen weiteren Sensor 30 auf. Der weitere Sensor 30 ist ein herkömmlicher ChemFET und weist eine nicht dargestellte Elektrode auf, welche dazu eingerichtet ist, ihr Potential zu ändern, wenn sie mit dem ersten Gas 4, beispielsweise Stickoxidgas, und/oder dem zweiten Gas 6, beispielsweise Kohlenwasserstoffgas, in Kontakt kommt. Der weitere Sensor 30 gemäß dem Ausführungsbeispiel nach Fig. 3 weist keinen Katalysator oder dergleichen auf, welcher geeignet wäre, das zweite Gas 6 derart zu verändern, dass dieses im Wesentlichen zu keiner Änderung des Potentials der Elektrode des weiteren Sensors 30 beiträgt. Der weitere Sensor 30 weist insbesondere keinen Katalysator auf, welcher geeignet wäre, Kohlenwasserstoffgas in Kohlendioxid und Wasser zu wandeln. Der weitere Sensor 30 ist also dazu eingerichtet, das erste und das zweite Gas 4; 6 zu detektieren.

Der Sensor 2 wird demnach einen Messwert für das erste Gas 4 erzeugen, während der weitere Sensor 30 einen Messwert für das erste und zweite Gas 4 und 6 erzeugen wird.

Der Chip 28 kann nun eine Auswerteeinheit 32 aufweisen, welche mit den Sensoren 2 und 30 signaltechnisch gekoppelt ist, angedeutet durch die Linien mit den Pfeilspitzen in Fig. 3, wobei die Auswerteeinheit 32 dazu eingerichtet ist, aus den vorgenannten Messwerten des Sensors 2 und des weiteren Sensors 30 auch den Anteil des zweiten Gases, also beispielsweise des Kohlenwasserstoffgases, in dem Abgasstrom 3 zu bestimmen.

Obwohl die Erfindung anhand von Ausführungsbeispielen konkret beschrieben wurde, ist sie darauf nicht beschränkt, sondern auf vielfältige Art und Weise modifizierbar.

## Patentansprüche

1. Verfahren zum Herstellen eines Sensors (2) zum Detektieren wenigstens eines ersten Gases (4) in einem Gasgemisch (3) aus wenigstens dem ersten und einem zweiten Gas (4; 6), aufweisend die folgenden Schritte:
Bereitstellen einer Elektrode (8), welche dazu eingerichtet ist, ihr Potential zu ändern, wenn sie mit dem ersten oder zweiten Gas (4; 6) oder beiden Gasen (4, 6) in Kontakt kommt; und
Anordnen eines Katalysators (16) beabstandet von der Elektrode (8) auf und/oder in einer durchgängig porösen Trägerkeramik (18), wobei der Katalysator (16) dazu eingerichtet ist, das zweite Gas (6) in dem Gasgemisch (3) chemisch derart zu verändern, dass das zweite Gas (6) zu keiner wesentlichen Veränderung des Potentials der Elektrode beiträgt, wenn das Gasgemisch (3) mit der Elektrode (8) in Kontakt kommt, **dadurch gekennzeichnet, dass** die Elektrode (8) zunächst mit einer Schutzschicht überzogen, daraufhin die Trägerkeramik (18), insbesondere bestehend aus Silizium oder Siliziumcarbid, auf der Schutzschicht erzeugt und die Schutzschicht anschließend wieder entfernt wird, sodass ein Spalt (24) zwischen der Elektrode (8) und der Trägerkeramik (18) entsteht.

2. Verfahren nach Anspruch 1, wobei die Schutzschicht mittels eines Lösungsmittels entfernt wird, welches durch die Poren in der porösen Trägerkeramik (18) hindurch mit der Schutzschicht in Kontakt gebracht wird.

3. Verfahren nach Anspruch 1, wobei die Schutzschicht dadurch entfernt wird, dass diese thermisch zersetzt wird und durch die Poren in der porösen Trägerkeramik (18) hindurch ausdampft.

## Claims

1. Method for producing a sensor (2) for detecting at least a first gas (4) in a gas mixture (3) comprising at least the first gas (4) and a second gas (6), having the following steps:
providing an electrode (8) which is set up to change its potential if it comes into contact with the first or second gas (4; 6) or both gases (4, 6); and
arranging a catalyst (16) at a distance from the electrode (8) on and/or in a fully porous carrier ceramic (18), the catalyst (16) being set up to chemically change the second gas (6) in the gas mixture (3) in such a manner that the second gas (6) does not contribute to a substantial change in the potential of the electrode if the gas mixture (3) comes into contact with the electrode (8), **characterized in that** the electrode (8) is first of all coated with a protective layer, the carrier ceramic (18), in particular consisting of silicon or silicon carbide, is then produced on the protective layer and the protective layer is then removed again, with the result that a gap (24) is produced between the electrode (8) and the carrier ceramic (18).

2. Method according to Claim 1, the protective layer being removed by means of a solvent which is brought into contact with the protective layer through the pores in the porous carrier ceramic (18).

3. Method according to Claim 1, the protective layer being removed by being thermally decomposed and evaporating through the pores in the porous carrier ceramic (18).

## Revendications

1. Procédé de fabrication d'un capteur (2) destiné à détecter au moins un premier gaz (4) dans un mélange de gaz (3) composé au moins du premier et d'un deuxième gaz (4; 6), comprenant les étapes suivantes:
préparer une électrode (8), qui est conçue de façon à modifier son potentiel lorsqu'elle vient en contact avec le premier ou le deuxième gaz (4; 6) ou avec les deux gaz (4, 6); et
disposer un catalyseur (16) à distance de l'électrode (8) sur et/ou dans une céramique de support poreuse perméable (18), le catalyseur (16) étant conçu de façon à modifier chimiquement le deuxième gaz (6) dans le mélange de gaz (3) d'une manière telle que le deuxième gaz (6) ne contribue à aucune modification importante du potentiel de l'électrode, lorsque le mélange de gaz (3) vient en contact avec l'électrode (8), **caractérisé en ce que** l'on recouvre d'abord l'électrode (8) avec une couche de protection, puis on produit sur la couche de protection la céramique de support (18) constituée notamment de silicium ou de carbure de silicium, et ensuite on enlève de nouveau la couche de protection, de telle manière qu'il se crée une fente (24) entre l'électrode (8) et la céramique de support (18).

2. Procédé selon la revendication 1, dans lequel on enlève la couche de protection au moyen d'un solvant, qui est mis en contact avec la couche de protection à travers les pores de la céramique de support poreuse (18).

3. Procédé selon la revendication 1, dans lequel on enlève la couche de protection en la décomposant thermiquement et en éliminant la vapeur à travers les pores de la céramique de support poreuse (18).
